# EUROPEAN PATENT APPLICATION

(11) **EP 2 461 112 A1**
(43) Date of publication of application: **06.06.2012**
(21) Application number: 10804188.0
(22) Date of filing: 31.05.2010
(51) Int. Cl.: F24F 7/00, A61L 9/22, F24F 1/00, F24F 13/20

(54) **AIR-CONDITIONING DEVICE**

(30) Priority: 31.07.2009 JP 2009179016
(71) Applicant: Sharp Kabushiki Kaisha, Osaka-shi, Osaka 545-8522 (JP)
(72) Inventor: KISHIMOTO, Kazuyuki, Osaka 545-8522 (JP); IIDA, Hiroyuki, Osaka 545-8522 (JP)
(74) Representative: Treeby, Philip David William
(86) International application number: PCT/JP2010/059185
(87) International publication number: WO 2011/013441

(57) **Abstract**

An air-conditioning device is provided that can generate ions without interfering with a flow of air blown out from an air outlet, and allows a user him/herself to easily change an ion generating electrode. Specifically, the air-conditioning device includes: a wind direction changing plate 30 provided in an air outlet that blows out air; and an ion generating unit 40 detachably mounted to the wind direction changing plate 30, wherein a recess 32 is formed in the wind direction changing plate 30, the recess 32 is formed to be larger than the ion generating unit 40 in one direction so that the ion generating unit 40 can be fitted in the recess 32 only in a predetermined position away from one of opposite ends of the recess 32 in one direction, a guide mechanism is provided that slidably holds the ion generating unit 40 fitted in the recess 32, and the guide mechanism slides the ion generating unit 40 fitted in the recess 32 toward one end in one direction to secure the ion generating unit 40 to the wind direction changing plate 30.

## Description

### Technical Field

The present invention relates to an air-conditioning device such as an air blower, an air conditioner, an air cleaner, a humidifier, a dehumidifier, a cooler, or a heater, each including a wind direction changing device in an air outlet that blows out air.

### Background Art

In recent years, value-added air conditioners including an ion generating device that generates positive and negative ions by discharge have come on the market. Positive and negative ions disappear when colliding with an obstacle while being sent by a flow of air.

Thus, an ion generating device is desirably mounted on a downstream side of a louver provided in an air outlet in an air conditioner, and an ion generating device provided slightly above the air outlet or an ion generating device including an ion generating electrode that generates ions on a louver surface as disclosed in Patent Document 1 is known. In particular, in Patent Document 1, a louver is located at a center of an air outlet, and thus the ions generated on the louver surface can be smoothly introduced into a flow of air, thereby increasing supply efficiency of positive and negative ions.

Patent Document 1: Japanese Patent Laid-Open No. 2004-347264

### Disclosure of the Invention

### Problems to be Solved by the Invention

Since an ion generating electrode applies a voltage to generate ions, a small amount of ions may be generated depending on use conditions (such as magnitude of the applied voltage or operating time). However, in Patent Document 1, the ion generating electrode and the louver are integrally formed, and it is difficult to actually replace an ion generating electrode.

In view of the above, the present invention has an object to provide an air-conditioning device that can generate ions without interfering with a flow of air blown out from an air outlet, and allows a user him/herself to easily replace an ion generating electrode.

### Means for Solving the Problems

In order to achieve the above object, the present invention provides an air-conditioning device including: a wind direction changing plate provided in an air outlet that blows out air; and an ion generating unit detachably mounted to the wind direction changing plate, wherein a recess is formed in the wind direction changing plate, and the ion generating unit is mounted so as to be fitted in the recess.

According to the above configuration, an ion generating electrode together with other components is housed in a casing and unitized, and the unit is mounted so as to be fitted in the recess formed in the wind direction changing plate. Thus, the air-conditioning device is easy to handle, and a user him/herself can replace the ion generating electrode.

Also, the ion generating unit is fitted in the recess, and thus the ion generating unit hardly interferes with a flow of air blown out from the air outlet. In this case, the wind direction changing plate is formed to be thick to form the recess, and the wind direction changing plate is formed to have a streamlined sectional shape to allow air to be smoothly fed to a distance without disturbing the flow of air blown out from the air outlet.

In the invention, the recess is formed to be larger than the ion generating unit in one direction so that the ion generating unit can be fitted in the recess only in a predetermined position away from one of opposite ends of the recess in one direction, a guide mechanism is provided that slidably holds the ion generating unit fitted in the recess, and the guide mechanism slides the ion generating unit fitted in the recess toward one end in one direction to secure the ion generating unit to the wind direction changing plate.

Specifically, the ion generating unit may be detached from the wind direction changing plate if being merely fitted in the recess. However, as in the above configuration, the ion generating unit fitted in the recess is slid perpendicularly to a fitting direction to prevent the ion generating unit from being detached.

The ion generating unit is preferably slid in parallel with a pivot direction of the wind direction changing plate. This prevents an influence of a pivot operation of the wind direction changing plate such as displacement of a sliding position of the ion generating unit.

Further, the ion generating unit fitted in the recess is slid toward one end in one direction, and a movement regulating member is inserted in a space created between an inner surface on the other end of the recess in one direction and the ion generating unit to regulate (lock) sliding of the ion generating unit, thereby reliably preventing the ion generating unit from being detached from the wind direction changing plate.

A method of inserting the movement regulating member in the recess space is not limited, and for example, the movement regulating member may be slidably provided so as to be advanced into and retracted from the space. The movement regulating member also may be provided so as to be pivotably switched between an insertion position for regulating movement of the ion generating unit and a release position for releasing regulation of the movement of the ion generating unit.

As such, the operation of the movement regulating member is the pivot operation different from the sliding operation of the ion generating unit, and thus an operator can easily visually recognize that the movement regulating member is in the release position. Thus, the movement of the ion generating unit can be reliably regulated while attention to switch the movement regulating member to the insertion position is drawn.

When the ion generating unit has not moved to a predetermined position, the movement regulating member hits the ion generating unit and cannot be placed in the insertion position. Thus, the movement regulating member also has a function of ensuring that the ion generating unit has moved to the predetermined position, by its being placed in the insertion position. In particular, as described later, connection of a connector can be reliably ensured in a case where the ion generating unit is moved to the predetermined position to electrically connect the connector.

The guide mechanism includes a protrusion formed on either opposite side surfaces of the ion generating unit in one direction or opposite inner surfaces of the recess in one direction, and a guide section that is formed on the other and slidably engages the protrusion, and the guide section includes a lateral guiding path provided in the one direction, and a vertical guiding path having one end connected to a midpoint of the lateral guiding path perpendicularly to the lateral guiding path, and the other end opening in a peripheral edge of the recess. In this case, the protrusion can be introduced through the vertical guiding path into the lateral guiding path. Specifically, the vertical guiding path is a guiding path for the protrusion when the ion generating unit is fitted in the recess, and the lateral guiding path is a guiding path for the protrusion when the ion generating unit is slid.

In the above configuration, when the ion generating unit is moved from one end toward the other end of the recess in one direction to abut against the other end with the protrusion being introduced into the lateral guiding path, the protrusion preferably passes over the vertical guiding path, and stops in the lateral guiding path on the side opposite to the side before movement when seen from the vertical guiding path.

Specifically, if the ion generating unit is merely fitted in the recess and secured, the vertical guiding path and the lateral guiding path may be connected into an L shape. However, with such a configuration, when the ion generating unit is detached from the recess and the ion generating unit is slid toward the other end of the recess to abut against the other end, the protrusion may slide in the vertical guiding path and the ion generating unit may be suddenly detached.

In contrast to this, in the present invention, the vertical guiding path is connected to the midpoint of the lateral guiding path, and thus when the protrusion abuts against the end of the lateral guiding path, the protrusion has passed over the vertical guiding path. Thus, the protrusion is kept held in the lateral guiding path and is not detached from the recess. This does not cause a situation in which the ion generating unit is suddenly detached and an operator panics.

In this case, to detach the ion generating unit from the recess, the ion generating unit moved in the lateral guiding path to abut against the other end in one direction is slightly returned toward one end, the protrusion is aligned with a connecting position of the vertical guiding path, and the protrusion is drawn out therefrom through the vertical guiding path. As such, in detaching the ion generating unit from the recess, operation can be safely performed even with careless handling. The detaching operation needs to be consciously performed, and thus the operator's attention can be drawn.

To generate ions from the ion generating unit, power needs to be supplied to the ion generating unit. Thus, the present invention adopts a configuration in which a pair of connectors that electrically connect the wind direction changing plate and the ion generating unit are formed on one end surface of the ion generating unit in one direction and an inner surface on one end of the recess in one direction, the connector includes a male connector and a female connector, and when the ion generating unit is slid toward one end of the recess in one direction, the male connector engages the female connector.

According to the above configuration, the connectors can be connected at the same time as the ion generating unit is slid in the recess to mount the ion generating unit to the wind direction changing plate. The movement regulating member described above is placed in the insertion position, thereby allowing the connectors to be secured in a connected state, and increasing safety.

In view of safety, for the connectors, it is preferable that the male connector is formed on one end surface of the ion generating unit in one direction, and the female connector is formed on the inner surface on one end of the recess in one direction. In this case, an electrode terminal is formed on a bottom surface rather than a top surface of the male connector, thereby avoiding a risk that a finger touches the electrode terminal and receives an electric shock in attaching or detaching the ion generating unit to and from the wind direction changing plate.

Further, if a configuration is adopted in which with the ion generating unit being fitted in the recess, a protective cover that prevents a finger from entering the male connector is formed on the top surface of the male connector, the risk of an electric shock can be more reliably avoided.

When the ion generating unit is fitted in the recess by the guide mechanism, it is sometimes difficult to find which position in the recess the ion generating unit should be aligned with. Thus, in the present invention, positioning indications are provided on both the ion generating unit and the recess so as to face each other when the ion generating unit is placed in a position to be fitted in the recess. The positioning indication may be, for example, a colored mark, or a solid indication such as irregularities.

The ion generating unit includes a casing, and an ion generating element housed in the casing, the casing includes a lower casing housed in the recess with the ion generating unit being mounted to the wind direction changing plate, and an upper casing protruding from a surface of the wind direction changing plate and having a space therein, the upper casing has openings on upstream and downstream sides in accordance with a direction of wind flowing along the surface of the wind direction changing plate, and the ion generating element generates ions in the space in the upper casing.

According to the above configuration, the ions are generated in the space in the upper casing, and also the generated ions can be introduced into the wind passing between the two openings and smoothly released to an outside. Specifically, the space in the upper casing has both a function as a space for generating ions and a function as a ventilation path.

The ion generating unit may generate, for example, either positive ions represented by H⁺(H₂O)ₘ (m is an arbitrary natural number) or negative ions represented by O₂⁻(H₂O)ₙ (n is an arbitrary natural number). One ion generating unit may alternately generate positive and negative ions from the same ion generating section, but ion generating units preferably separately generate positive and negative ions.

When a positive ion generating section and a negative ion generating section are separately formed as ion generating units, the ion generating units are preferably placed with a space therebetween in parallel with the direction of wind flowing along the wind direction changing plate. This can prevent positive and negative ions generated from the ion generating units from contacting and reacting with each other and disappearing. The ion generating unit may generate negative ions.

The wind direction changing plate described above may be, specifically, a wind guide panel provided on a front side of the air outlet as a part of a cabinet. In the air conditioner including the wind guide panel, the wind direction changing plate may be an auxiliary louver that changes a vertical angle according to an position of the wind guide panel, and changes a vertical wind direction while straightening the wind blown out from the air outlet, and the ion generating unit may be provided on the auxiliary louver.

The wind direction changing plate in the present invention may be at least one of a group of a plurality of louvers provided in an air outlet of a conventional air conditioner, and the ion generating unit may be provided on the louver.

The air-conditioning device according to the present invention may include, specifically, an air blower, an air conditioner, a humidifier, a dehumidifier, an air cleaner, a cooler, a heater, or the like.

### Effects of the Invention

As described above, according to the present invention, the recess is formed in the wind direction changing plate, and the ion generating unit is mounted so as to be fitted in the recess. This allows ions to be generated without interfering with a flow of air blown out from the air outlet, and allows a user him/herself to easily replace the ion generating electrode.

### Brief Description of the Drawings

Figure 1 is a perspective view of an indoor unit of an air conditioner according to an embodiment of the present invention;
Figure 2 is a schematic sectional view of the indoor unit when a wind guide panel is closed;
Figure 3 is a perspective view of the indoor unit when the wind guide panel is opened downward;
Figure 4 is a schematic sectional view of the indoor unit when the wind guide panel is opened upward;
Figure 5 is a perspective view of an auxiliary louver;
Figure 6 is a partial perspective view showing a state where an ion generating unit is detached from the auxiliary louver;
Figure 7 is a partial perspective view showing a state where the ion generating unit is fitted in the auxiliary louver;
Figure 8 is a partial perspective view showing a state where the ion generating unit fitted in the auxiliary louver is slid and secured;
Figure 9 is a partially enlarged sectional view showing a connector portion in Figure 7;
Figure 10 is a partially enlarged sectional view showing a connector portion in Figure 8;
Figure 11 is a partial perspective view showing a state where a movement regulating member is placed in a regulating position;
Figure 12 is a partial perspective view showing a state where the ion generating unit is moved to abut in a detaching direction;
Figure 13 shows a function block of the ion generating device;
Figure 14 is an exploded perspective view of an ion generating element;
Figure 15 is a schematic plan view showing the ion generating unit with an upper casing being removed; and
Figure 16 is a schematic plan view showing an ion generating unit of a different aspect from that in Figure 15.

### Description of Symbols

- 1: heat exchanger
- 2: indoor fan
- 3: cabinet
- 4: air inlet
- 5: air outlet
- 6: air passage
- 7: filter
- 8: cleaning device
- 9: dust removing section
- 10: filter moving path
- 20: wind guide panel
- 21: front panel
- 22: lower pivot
- 23: upper pivot
- 24: vertical louver
- 30: auxiliary louver
- 31: rotating shaft
- 32: recess
- 33: positioning indication
- 34: protrusion
- 35: guide section
- 36: vertical guiding path
- 37: lateral guiding path
- 38: movement regulating member
- 40: ion generating unit
- 41: ion generating element
- 42: casing
- 47: male connector
- 48: female connector
- 49: protective cover
- 51: induction electrode
- 52: discharge electrode
- 53: substrate
- 55: positive ion generating section
- 56: negative ion generating section
- 57: ventilation path

### Mode for Carrying Out the Invention

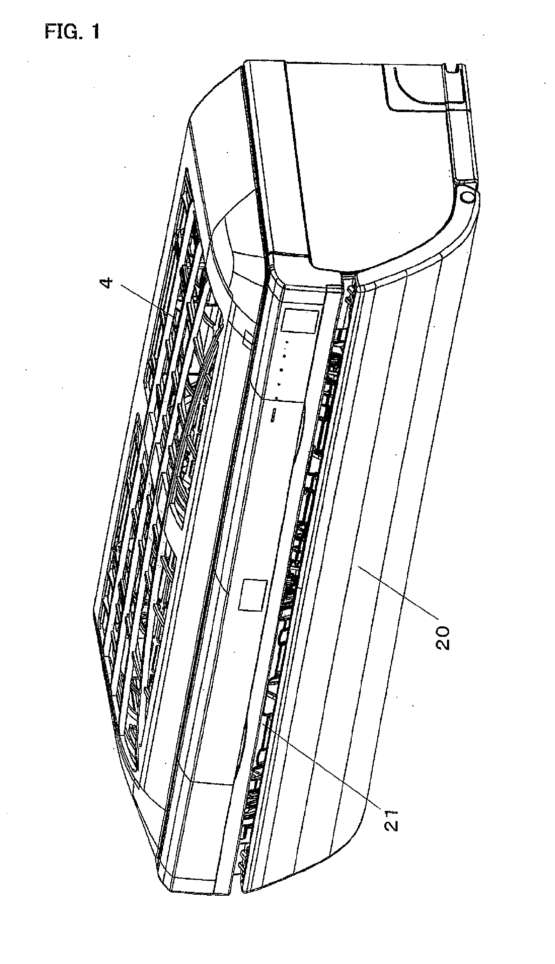

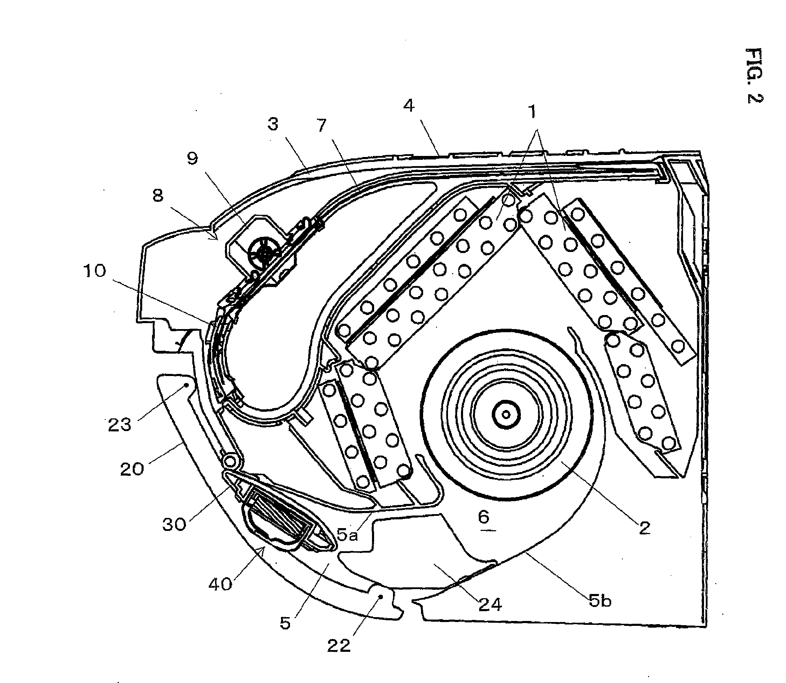

Now, an embodiment of the present invention will be described with reference to the drawings. In this embodiment, a case will be described where an indoor unit of a separate air conditioner mainly having cooling and heating functions is used as an air-conditioning device. Figure 1 is an appearance perspective view of an indoor unit of an air conditioner, and Figure 2 is a sectional view of the indoor unit in Figure 1.

The indoor unit includes a heat exchanger 1 and an indoor fan 2, which are housed in a cabinet 3. The cabinet 3 has a curved surface from a front surface to a bottom surface. An air inlet 4 is formed in an upper surface of the cabinet 3, and an air outlet 5 is formed in the curved surface.

In the cabinet 3, an air passage 6 extending from the air inlet 4 to the air outlet 5 is formed, and the heat exchanger 1 and the indoor fan 2 are provided in the air passage 6. A filter 7 is provided between the air inlet 4 and the heat exchanger 1 to remove dust from indoor air sucked from the air inlet 4. A cleaning device 8 that cleans the filter 7 is provided.

The cleaning device 8 moves the filter 7 in the cabinet 3 to pass through a dust removing section 9, and remove dust adhering to the filter 7 in the dust removing section 9. On the front side in the cabinet 3, a filter moving path 10 curved into a U shape in side view is formed, and a moving section including a motor and gears reciprocates the filter 7 along the filter moving path 10. In the dust removing section 9, a rotating brush scrapes dust off the passing filter 7, and a suction fan blows air substantially in parallel with the filter 7 (in a lateral direction) to suck and discharge the scraped dust.

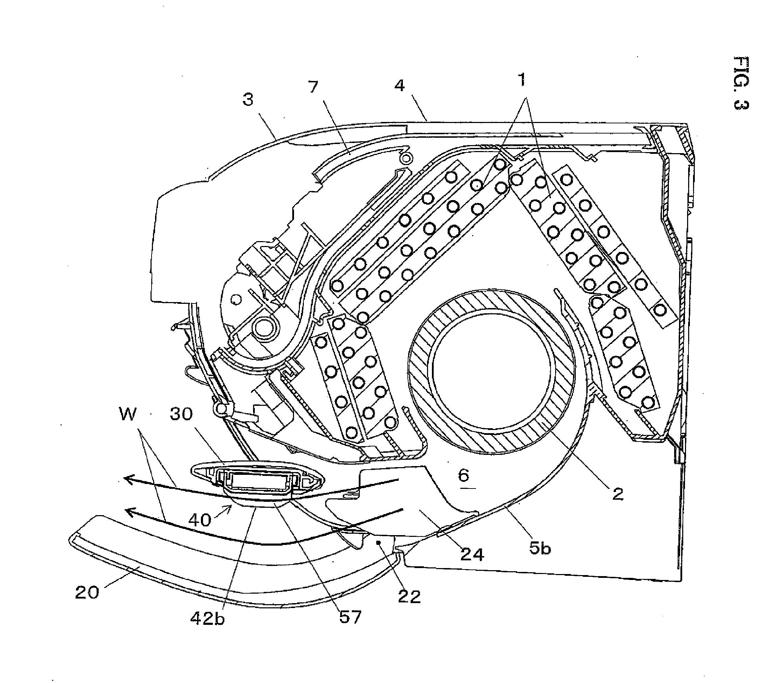

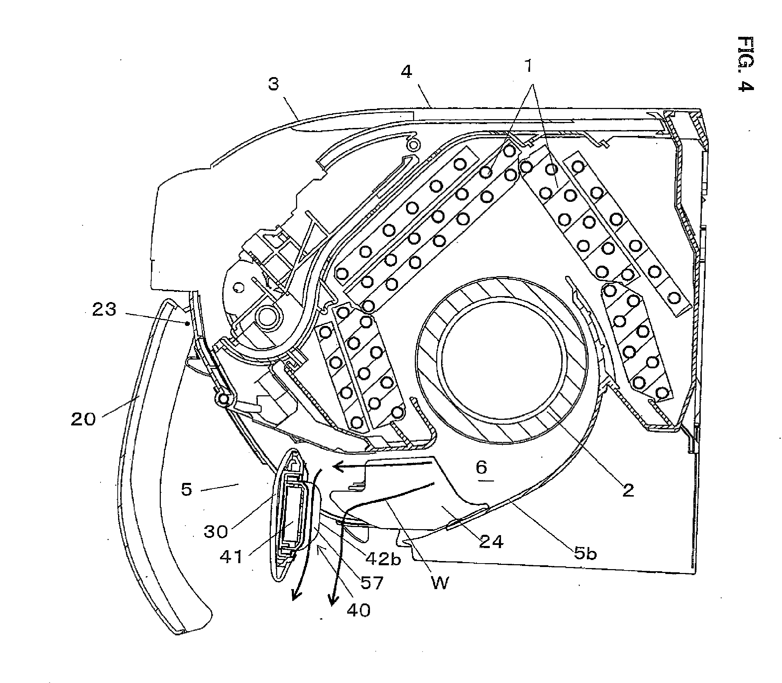

A wind guide panel 20 that opens/closes the air outlet 5 is provided on the curved surface of the cabinet 3. As shown in Figures 3 and 4, the wind guide panel 20 can be opened upward and downward and is removable from the cabinet 3. Such a configuration is known, and can be achieved by adopting a mechanism disclosed in, for example, Japanese Patent Laid-Open No. 2009-63258.

The wind guide panel 20 is formed of one curved panel, and has a width equal to that of the cabinet 3 and larger than that of the air outlet 5. On the front surface of the cabinet 3, a front panel 21 is formed from a middle in the front surface toward the bottom surface in a one level lower position. Thus, a recessed portion is formed in an entire width direction, and the wind guide panel 20 is fitted in the recessed portion. An opening is formed in the front panel 21 that forms the recessed portion, and the opening is the air outlet 5. Thus, the wind guide panel 20 is placed forward of the air outlet 5, and covers the air outlet 5 and the front panel 21 around the air outlet 5. At this time, the wind guide panel 20 is placed in a closed position shown in Figure 2.

The wind guide panel 20 has an outer surface that forms a smooth curved surface extending from the front surface to the bottom surface of the cabinet 3. Specifically, the wind guide panel 20 is a member that constitutes a part of the front surface of the cabinet 3. In other words, a part of a panel of the cabinet 3 is used as the wind guide panel 20. Thus, the wind guide panel 20 is a long panel having a longer entire length than a louver used in a conventional air conditioner.

The wind guide panel 20 is pivoted in different directions around upper and lower pivots and is opened upward or downward. An upper pivot 23 and a lower pivot 22 are formed in parallel with a lateral direction of the cabinet 3. As shown in Figure 3, the wind guide panel 20 is opened downward around the lower pivot 22 in a cooling operation. In this downwardly opened position, the wind guide panel 20 is connected to a lower wall of the air outlet 5, and the wind guide panel 20 and an upper wall of the air outlet 5 constitute a long nozzle. The wind guide panel 20 guides cool air obliquely upward, and the cool air blows out along a ceiling.

As shown in Figure 4, in a heating operation, the wind guide panel 20 is opened upward around the upper pivot 23. In this upwardly opened position, the wind guide panel 20 closes the front side of the air outlet 5, holds warm air blown out forward and guides the warm air toward a floor surface. Also in an initial stage of the cooling operation, the wind guide panel 20 is placed in the upwardly opened position, and cool air is blown out toward the floor surface for rapid cooling. As shown in Figure 2, the wind guide panel 20 is placed in a closed position when the operation is stopped, covers the air outlet 5, and is integrated with the cabinet 3.

In this embodiment, a vertical louver 24 and also an auxiliary louver 30 are provided in the air outlet 5. The vertical louver 24 changes its lateral angle to change a lateral direction of wind. The auxiliary louver 30 is provided at an outlet portion of the air outlet 5 on a front side of the vertical louver 24, changes its vertical angle according to the position of the wind guide panel 20 to straighten the wind and change a vertical direction of wind W blown out from the air outlet 5. In this embodiment, the auxiliary louver 30 is a wind direction changing plate in the present invention, and an ion generating unit 40 is detachably mounted to the auxiliary louver 30. The auxiliary louver 30 will be now described in detail.

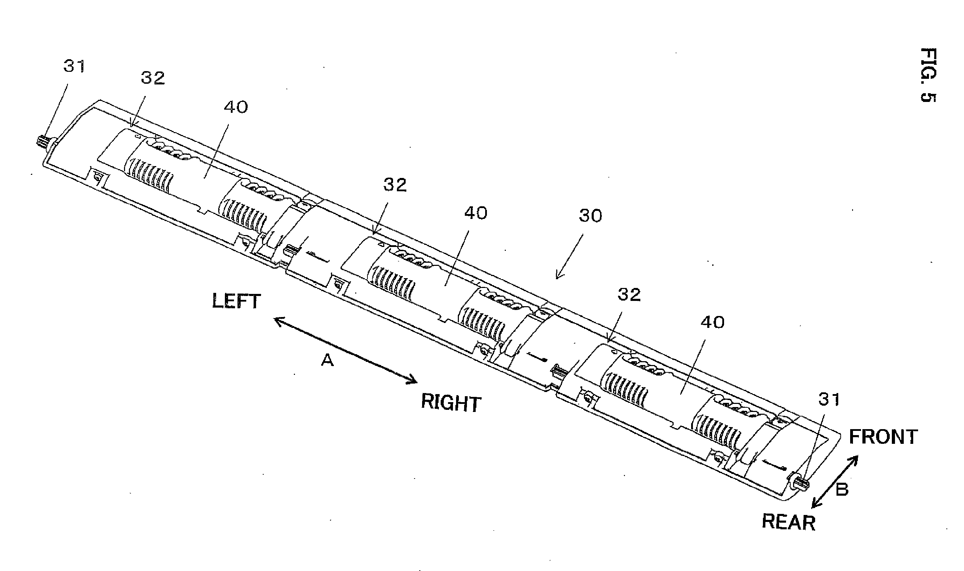

As shown in Figure 5, the auxiliary louver 30 is formed to have a long plate shape in a lateral direction A and tapered front and rear ends. Specifically, the auxiliary louver 30 has a streamlined sectional shape in a fore/aft direction B. On a rear end side (base end side) of the auxiliary louver 30, a rotating shaft 31 having an axial direction in the lateral direction is secured to shaft connecting sections formed at left and right ends. The rotating shaft 31 is placed on an upper wall 5a of upper and lower walls 5a and 5b that form an air passage 6 leading to the air outlet 5, passes through left and right side walls of the air outlet 5, and is rotatably bearing mounted. An unshown louver motor is connected to an end of the rotating shaft 31, and the rotating shaft 31 is rotatable by the louver motor.

In a lower surface of the auxiliary louver 30, three recesses 32 are formed at intervals in the lateral direction, and an ion generating unit 40 is detachably fitted in each recess 32. The ion generating unit 40 fitted in the recess 32 is held by the guide mechanism slidably between one and the other ends of the recess 32 in one direction. A structure of the auxiliary louver 30 including the ion generating unit 40 will be now described in detail. In Figures 5 to 12, a lower surface of the auxiliary louver is shown upward. In this embodiment, the plurality of recesses 32 are formed in the auxiliary louver 30, but one recess 32 may be formed.

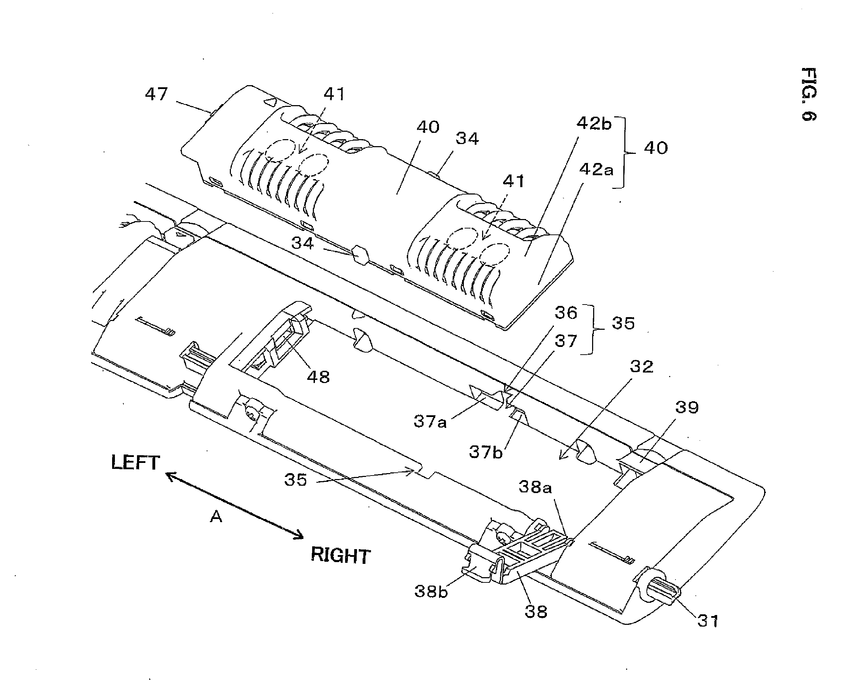

As shown in Figure 6, the recess 32 has a longer length in the lateral direction A than the ion generating unit 40, and substantially the same length in the fore/aft direction B as the ion generating unit 40. Thus, when the ion generating unit is fitted in the recess 32, only a slight gap is created therebetween in the fore/aft direction B but a considerable space is created therebetween in the lateral direction A.

The ion generating unit 40 fitted in the recess 32 is slidable in the lateral direction A using the space created in the lateral direction A. Specifically, in this embodiment, the lateral direction A in the Figures 5 to 12 is a sliding direction (one direction) of the ion generating unit 40, and of opposite ends of the recess in the lateral direction, a left end is one end in one direction, and a right end is the other end in one direction.

The ion generating unit 40 can be fitted and secured in the recess 32 by the guide mechanism. The guide mechanism includes a pair of protrusions 34 and 34 provided on opposite side surfaces formed in the lateral direction (sliding direction) A in the ion generating unit 40, and guide sections 35 that are formed in opposite inner surfaces of the recess 32 in the lateral direction A, and slidably engage the protrusions 34.

The guide section 35 includes a vertical guiding path 36 extending in a recess depth direction from a peripheral edge of the recess, and a lateral guiding path 37 connected perpendicularly (in the lateral direction A) to the vertical guiding path 36 on a back side of the vertical guiding path 36. The vertical guiding path 36 and the lateral guiding path 37 are formed to be recessed in the inner surface of the recess 32. More specifically, an upper end of the vertical guiding path 36 is exposed to the peripheral edge of the recess 32, and a recessed opening is formed therein. The protrusion 34 can be introduced into the vertical guiding path 36 through the opening.

The lateral guiding path 37 includes a first lateral guiding path 37a extending from the vertical guiding path 36 to left in the lateral direction A, and a second lateral guiding path 37b extending from the vertical guiding path 36 to right in the lateral direction A, which is opposite to the direction of the first lateral guiding path 37a. Specifically, the vertical guiding path 36 is formed to be connected to a midpoint of the lateral guiding path 37 formed in the lateral direction A.

The vertical guiding path 36 is formed so that an opening position at the upper end of the vertical guiding path 36 matches a position of the protrusion 34 when the ion generating unit 40 is in a position away from a left inner surface of the recess 32 (a position close to a right inner surface of the recess 32 in this embodiment). The ion generating unit 40 is pressed into the recess 32 in that position, and thus the protrusion 34 is introduced through the vertical guiding path 36 into the lateral guiding path 37.

The forming position of the vertical guiding path 36 is not limited to as long as the ion generating unit 40 is in a position away from the left inner surface of the recess 32. Thus, for example, when the ion generating unit 40 is positioned at the center of the recess 32 in the lateral direction, the position of the protrusion 34 can match the position of the opening of the vertical guiding path 36. In order to minimize the length of the recess 32 in the lateral direction A, the position of the protrusion 34 preferably matches the position of the opening of the vertical guiding path 36 when the ion generating unit 40 is in a position close to the right inner surface of the recess 32.

A pair of connectors 47 and 48 for supplying power to the ion generating unit are provided on a left end surface of the ion generating unit 40 and the left inner surface of the recess 32. More specifically, a male connector 47 for inputting power is provided on a left end surface of the ion generating unit 40, and a female connector 48 for supplying power is provided on the left inner surface of the recess 32. The female connector 48 is connected to a power supply mounted in the cabinet 3 via a lead wire. The lead wire is routed from the cabinet 3 through the rotating shaft 31 into the auxiliary louver 30. In this embodiment, the first lateral guiding path 37a extends toward the female connector 48.

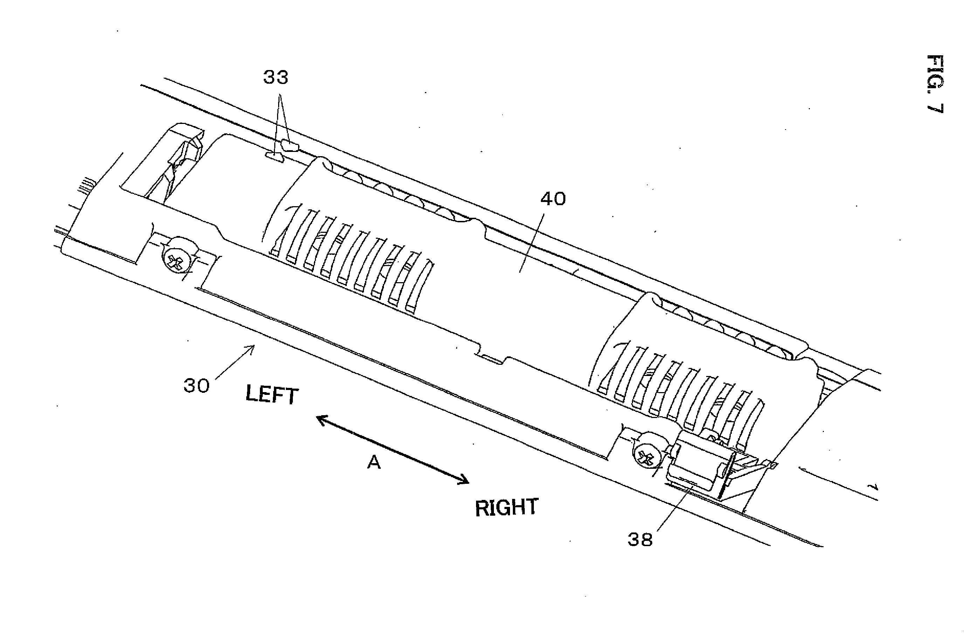

When the ion generating unit 40 is secured to the recess 32 in the guide mechanism having the above configuration, first as shown in Figure 7, the protrusion 34 is placed on the position of the vertical guiding path 36 of the guide section 35 to insert the ion generating unit 40 into the recess 32. The ion generating unit 40 and the peripheral edge of the recess 32 have triangular positioning indications 33 and 33 so as to face each other when the position of the protrusion 34 matches the position of the vertical guiding path 36.

The positioning indications 33 and 33 are formed protrudedly, and can be formed at the same time as a casing of the ion generating unit 40 or the auxiliary louver 30 is formed.

When the ion generating unit 40 is fitted in the recess 32, the protrusion 34 is guided through the vertical guiding path 36 to the lateral guiding path 37. When the ion generating unit 40 is slid from this state to the left in the lateral direction A, the protrusion 34 slides in the first lateral guiding path 37a. As such, vertical movement of the protrusion 34 is regulated by the first lateral guiding path 37a, and thus the ion generating unit 40 is secured to the auxiliary louver 30 and prevented from being detached. When the ion generating unit 40 reaches the left end of the recess 32, the male connector 47 engages the female connector 48.

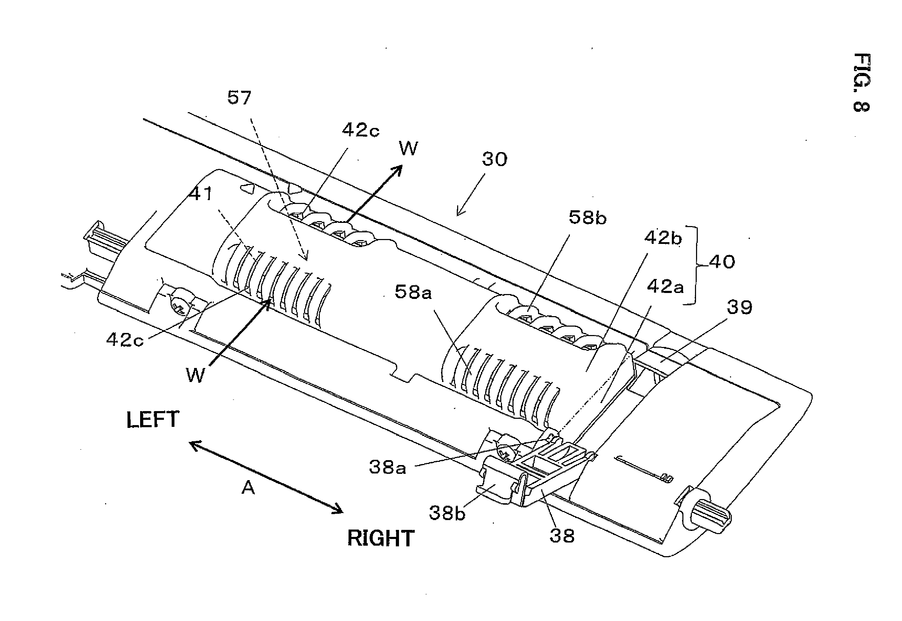

As shown in Figure 8, when the ion generating unit 40 is moved to the left end of the recess 32, a space is created between a right end surface of the ion generating unit 40 and a right inner surface of the recess 32 at the right end of the recess 32. In the present invention, this space is filled with a movement regulating member 38 to regulate movement of the ion generating unit 40.

Specifically, the movement regulating member 38 is provided to face the recess 32 at the right end of the recess 32. The movement regulating member 38 is rotatable around a rotating shaft 38a having an axial direction parallel to the lateral direction A in a position close to a rear inner surface of the recess 32. The movement regulating member 38 is provided so as to be pivotably switched between an insertion position where the movement of the ion generating unit 40 is regulated and a release position where regulation of the movement of the ion generating unit is released.

In Figure 8, the movement regulating member 38 is in the release position. As shown in Figure 8, when the movement regulating member 38 is in the release position, the movement regulating member 38 is raised from the auxiliary louver 30, and thus the movement regulating member 38 being in the release position can be easily visually recognized, thereby preventing forgetting to switch to the regulation position.

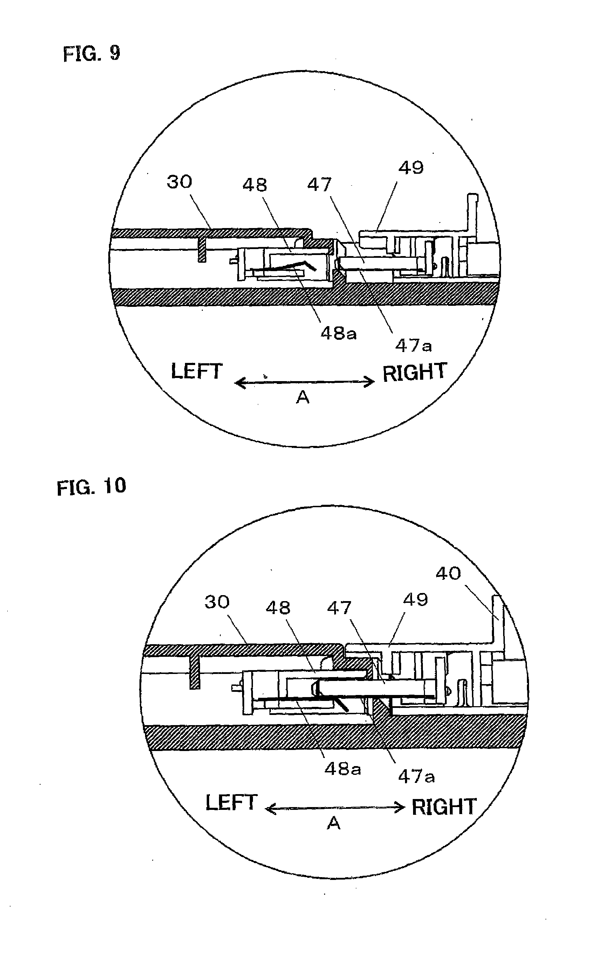

Figures 9 and 10 are partially enlarged sectional views showing the connectors 47 and 48 in Figures 7 and 8. As shown, an electrode terminal 47a of the male connector 47 is formed on a bottom surface rather than a top surface of the connector, thereby avoiding a risk that a finger touches the electrode terminal 47a and receives an electric shock. An electrode terminal 48a of the female connector 48 is formed on the bottom surface in the connector.

On the top surface side (upper side) of the male connector 47, a protective cover 49 is formed that prevents a finger from entering the male connector 47, and the risk of an electric shock can be more reliably avoided. The protective cover 49 is formed so that when the ion generating unit 40 is slid to the left in the recess 32 and approaches a position where the male connector 47 engages the female connector 48, the protective cover 49 reaches the surface of the auxiliary louver housing the female connector 48. Thus, when the male connector 47 is energized, a gap between the ion generating unit 40 and the left inner surface of the recess 32 is eliminated to prevent a finger from entering the male connector 47.

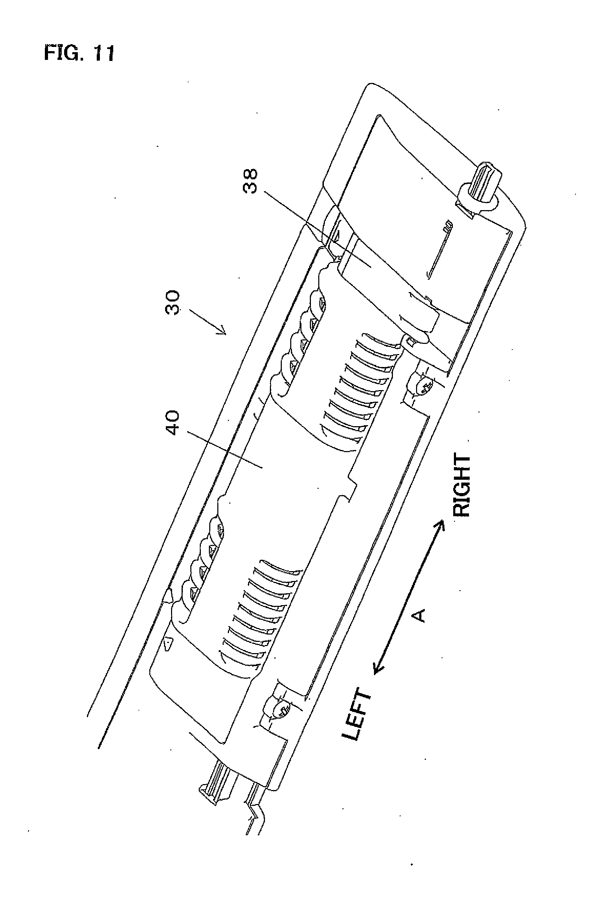

After the ion generating unit 40 is slid to the left end of the recess 32 and the connectors 47 and 48 engage each other, as shown in Figure 11, the movement regulating member 38 is pivoted to the regulation position to regulate sliding of the ion generating unit 40. This can ensure connection of the connectors 47 and 48.

An elastic hook 38b is formed at an end of the movement regulating member 38, and a receiving section 39 of the elastic hook 38b is formed in a position facing the movement regulating member 38 on a front inner surface of the recess 32. When the movement regulating member 38 is placed in the regulation position, the elastic hook 38b engages the receiving section 39, and the movement regulating member 38 maintains the regulation position.

When the ion generating unit 40 is detached from the auxiliary louver 30, the elastic hook 38b is disengaged from the receiving section 39 to bring the movement regulating member 38 into the release position, and then the ion generating unit 40 is slid to the right side in the lateral direction A to the position of the vertical guiding path 36 (the same state in Figure 7). The ion generating unit 40 is raised toward the surface in that position, and thus the protrusion 34 is disengaged from the vertical guiding path 37, and the ion generating unit 40 can be detached from the auxiliary louver 30.

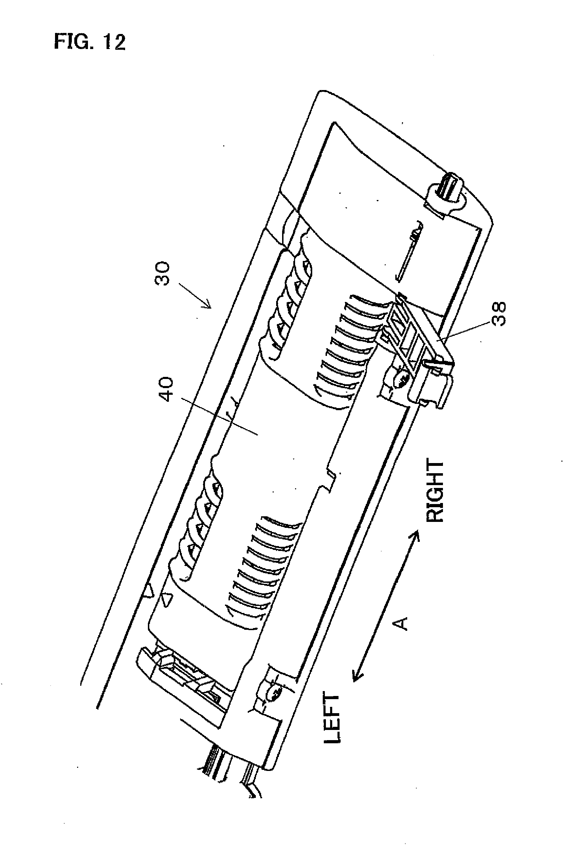

At this time, if an inexperienced operator moves the ion generating unit 40 so that the protrusion 34 abuts against the right end of the lateral guiding path 37, as shown in Figure 12, the protrusion 34 passes over the vertical guiding path 36 and stops in the second lateral guiding path 37b on the side opposite to the first lateral guiding path 37a. This prevents the ion generating unit 40 from being unexpectedly detached from the recess 32.

In this case, to detach the ion generating unit from the recess, the ion generating unit 40 may be slightly moved back to the left, the protrusion 34 may be aligned with a connecting position of the vertical guiding path 36, and the protrusion 34 may be drawn out therefrom through the vertical guiding path. The above configuration is particularly useful when the air-conditioning device is placed in an indoor upper position, and an operator looks up and detaches the ion generating unit.

As such, the vertical guiding path 36 is connected to the midpoint of the lateral guiding path 37, and thus when the protrusion 34 abuts against the end of the lateral guiding path 37, the protrusion 34 has passed over the vertical guiding path 36. Thus, the protrusion 34 is held in the lateral guiding path 37 and not detached from the recess 32.

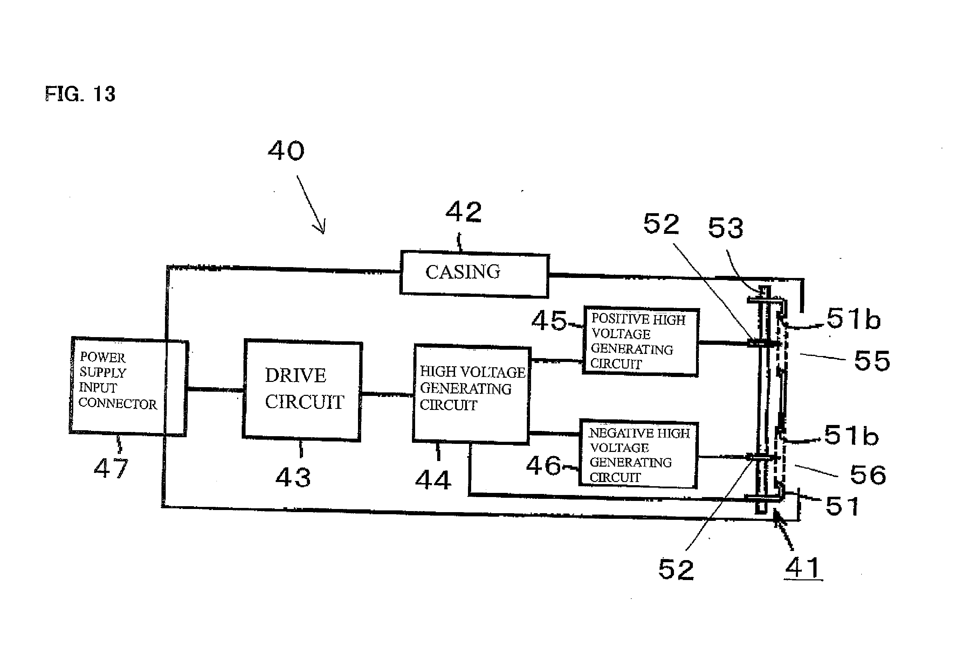

Next, the configuration of the ion generating unit 40 will be described in detail. As shown in Figure 13, the ion generating unit 40 includes an ion generating element 41, a power supply input connector 47, a drive circuit 43, a high voltage generating circuit 44, a positive high voltage generating circuit 45, and a negative high voltage generating circuit 46, which are housed in a casing 42.

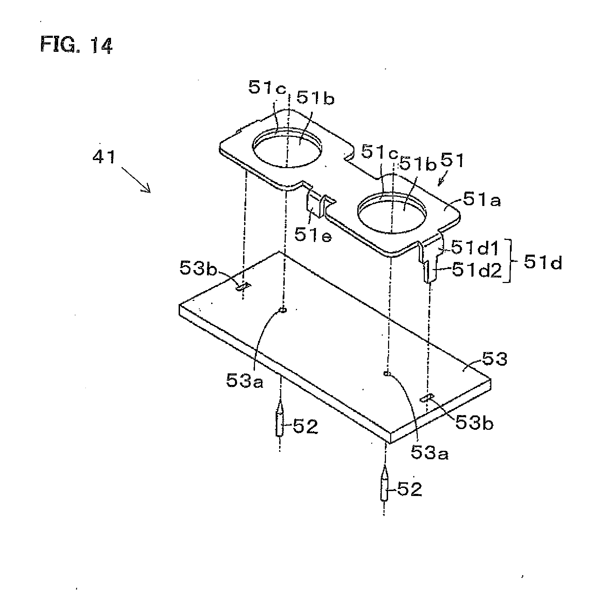

As shown in Figure 14, the ion generating element 41 includes an induction electrode 51, discharge electrodes 52, and a substrate 53. The discharge electrode 52 has a needle-like tip. The substrate 53 has through holes 53a through which the discharge electrodes 52 are inserted, and through holes 53b through which insertion portions 51d2 of a substrate insertion section 51d are inserted.

The needle-like discharge electrodes 52 are inserted or press-fitted into the through holes 53a and passed through and supported by the substrate 53. Thus, one needle-like end of the discharge electrode 52 protrudes on a front surface of the substrate 53, and to the other end protruding on a back surface of the substrate 53, a lead wire or a wiring pattern can be electrically connected by solder 54.

The insertion portions 51d2 of the induction electrode 51 are inserted into the through holes 53b and passed through and supported by the substrate 53. To a tip of the insertion portion 51d2 protruding on a back surface of the substrate 53, a lead wire or a wiring pattern can be electrically connected by solder 54.

With the induction electrode 51 being supported by the substrate 53, a step between a support portion 51d1 and the insertion portion 51d2 abuts against the front surface of the substrate 53. Thus, a top section 51a of the induction electrode 51 is supported at a predetermined distance from the substrate 53. A tip of a substrate support section 51e of the induction electrode 51 secondarily abuts against the front surface of the substrate 53. Specifically, the induction electrode 51 can be positioned in a thickness direction of the substrate 53 using the substrate insertion section 51d and the substrate support section 51e.

With the induction electrode 51 being supported by the substrate 53, the discharge electrode 52 is placed so that a needle-like tip thereof is positioned at the center of the circular through hole 51b, and positioned within a range of a thickness of a peripheral edge of the through hole 51b (that is, a bending length of a bent section 51c).

In order to release both positive and negative ions, a needle-like tip position of the discharge electrode 52 that generates positive ions and a needle-like tip position of the discharge electrode 52 that generates negative ions are placed with a predetermined distance therebetween, the tips are aligned with the center of the through holes 51b in the induction electrode 51, and placed within the thickness range of the through holes 51b in the induction electrode 51 so that the induction electrode 51 and the needle-like tips of the discharge electrodes 52 face each other with an air space therebetween.

In the ion generating element 41, the plate-like induction electrode 51 and the needle-like discharge electrodes 52 are placed with the predetermined distance therebetween as described above, and a high voltage is applied to between the induction electrode 51 and the discharge electrodes 52. Then, corona discharge occurs at the tips of the needle-like discharge electrodes 52. The corona discharge generates at least either positive ions or negative ions, and the ions are released from the through hole 51b provided in the induction electrode 51 to an outside of the ion generating element 41. Further, air is blown to allow the ions to be more effectively released.

The positive ion is a cluster ion which is a hydrogen ion(H⁺) with a plurality of water molecules around the hydrogen ion, and represented as H⁺(H₂O)ₘ (m is an arbitrary natural number). The negative ion is a cluster ion which is a oxygen ion(O₂⁻) with a plurality of water molecules around the oxygen ion, and represented as O₂⁻(H₂O)ₙ (n is an arbitrary natural number).

Both the positive and negative ions are released, and thus H⁺(H₂O)ₘ (m is an arbitrary natural number) as positive ions in the air and O₂⁻(H₂O)ₙ (n is an arbitrary natural number) as negative ions substantially of the same amount are generated. Thus, the positive and negative ions surround mold or viruses suspended in the air, and can remove the suspended mold or the like by action of a hydroxide radical (• OH) of activated species generated at that time.

In the ion generating device, positive corona discharge is caused at the tip of one discharge electrode 52 to generate positive ions, and negative corona discharge is caused at the tip of the other discharge electrode 52 to generate negative ions. Any waveform may be herein applied, and a waveform of a high voltage such as a DC, an AC waveform biased to positive or negative, or a pulse waveform biased to positive or negative may be applied. A voltage value is selected within a voltage range sufficient for causing discharge and generating predetermined ion species.

The ion generating device may generate other ion species, not limited to the ion species in this embodiment, and may generate, for example, negative ions.

To the power supply input connector 47, DC power or commercial AC power as input power is supplied through the connector 48. The drive circuit 43 to which the input voltage is supplied through the power supply input connector 47 drives the high voltage generating circuit 44 to increase the input voltage to generate high voltage. One end of the high voltage generating circuit 44 is electrically connected to the induction electrode 51. The high voltage generating circuit 44 applies a high voltage of positive polarity to the induction electrode 51 through the positive high voltage generating circuit 45 to the needle-like discharge electrode 52 that generates positive ions, and applies a high voltage of negative polarity to the induction electrode 51 through the negative high voltage generating circuit 46 to the needle-like discharge electrode 52 that generates negative ions.

In the casing 42, the drive circuit 43, the high voltage generating circuit 44, the positive high voltage generating circuit 45, and the negative high voltage generating circuit 46 are provided at the center thereof, and the ion generating elements 41 are provided on left and right sides thereof. Specifically, the drive circuit 43, the high voltage generating circuit 44, the positive high voltage generating circuit 45, and the negative high voltage generating circuit 46 are shared, and two needle-like electrodes are connected to each of the positive high voltage generating circuit 45 and the negative high voltage generating circuit 46.

As shown in Figure 8, with the ion generating unit 40 being mounted to the auxiliary louver 30, the casing 42 of the ion generating unit 40 includes a lower casing 42a housed in the recess 32, and an upper casing 42b protruding from the surface of the auxiliary louver 30 and having a space therein with reference to an opening position of the recess 32 (a position shown by a dash-double-dot line in the drawing).

The upper casing 42b has openings 42c and 42c formed on upstream and downstream sides of wind W flowing along the surface of the auxiliary louver 30, and a space in the upper casing 42b is a ventilation path 57 through which the wind W passes. The ion generating element 41 is provided to face the ventilation path 57 in the lower casing 42a.

In the above configuration, ions generated from the ion generating element 41 are diffused into the air in the ventilation path 57, and introduced into the wind W and smoothly released to the outside when the wind W blown out from the air outlet 5 passes through the ventilation path 57.

In the opening 42c, a guard member 58 is provided that guards a finger from entering the ventilation path 57. The guard member 58 in this embodiment is elongated vertical bars. The guard member 58 has tapered front and rear ends, and has a shape with low air resistance so as to minimize disturbance of a flow of wind flowing in the ventilation path 57.

In this embodiment, a gap between windward vertical bars 58a is narrow, and a gap between leeward vertical bars 58b is wide. This is to prevent a finger from touching the discharge electrode 52 for safety because the discharge electrode 52 of the ion generating element 41 is closer to the windward vertical bars 58a than the leeward vertical bars 58b. Further, the windward vertical bars 58a has a function of reducing strength of the wind W introduced into the ventilation path 57. This allows ions generated from the ion generating element 41 to be sufficiently diffused into the air in the ventilation path 57.

Meanwhile, the leeward vertical bars 58b has a wide space of the vertical bars 58b so as not to interfere with generated ions. This can provide advantages of safety and preventing generated ions from abutting against the vertical bars 58b and disappearing.

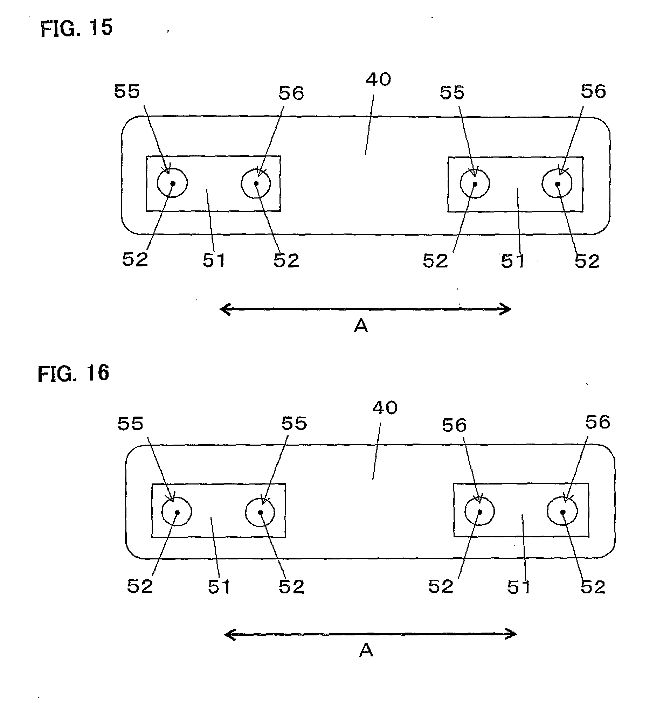

As shown in Figure 13, the discharge electrode 52 connected to the positive high voltage generating circuit 45 is a positive ion generating section 55 that generates positive ions, and the discharge electrode 52 connected to the negative high voltage generating circuit 46 is a negative ion generating section 56 that generates negative ions. Figure 15 is a schematic plan view of the ion generating unit with the upper casing 42b being removed. As shown, in this embodiment, the positive ion generating section 55 and the negative ion generating section 56 are formed with a space therebetween in the lateral direction A. This can prevent a reduction in the number of ions being carried in a flow of air.

As another aspect of arrangement of the discharge electrodes 52, as shown in Figure 16, the positive ion generating sections 55 and the negative ion generating sections 56 may be separately provided at left and right of the casing 42. Specifically, in this aspect, two discharge electrodes 52 connected to the positive high voltage generating circuit 45 and two discharge electrodes 52 connected to the negative high voltage generating circuit 46 are collectively placed either at the left or right. This aspect is particularly useful when the casing may have a sufficiently long lateral length. Thus, the left and right ion generating sections can be separated as much as possible, thereby more efficiently preventing the positive and negative ions from contacting and disappearing.

In the air conditioner, an unshown outdoor unit is provided outdoor with respect to an indoor unit. The outdoor unit houses a compressor, a heat exchanger, a four-way valve, an outdoor fan, or the like, and these components and the heat exchanger 1 in the indoor unit constitute a refrigeration cycle. A control section that controls driving of the refrigeration cycle, the wind guide panel 20, the auxiliary louver, or the like is provided in the indoor unit.

The control section constituted by a microcomputer controls the refrigeration cycle based on a user's instructions and detection signals of various sensors such as temperature sensors that detects a room temperature or an outside air temperature, and performs cooling and heating operations. At this time, the control section controls an opening/closing mechanism according to the cooling/heating operation to open/close the wind guide panel 20, and drives the auxiliary louver 30 according thereto. The control section controls the cleaning device 8 to clean the filter 7 regularly or according to a user's instructions.

An operation of the auxiliary louver 30 will be described in detail. As shown in Figure 2, the auxiliary louver 30 can be housed in a space in the air outlet 5 in an upward position in contact with the upper wall 5a, and the wind guide panel 20 can be closed.

If the wind guide panel 20 is detached in the closed position of the wind guide panel 20, the upper casing 42b of the ion generating unit 40 is directed downward. Thus, the ion generating unit 40 can be replaced without moving the auxiliary louver 30. Depending on models of the air-conditioning device, if the wind guide panel 20 is removed in the closed position of the wind guide panel 20, the upper casing 42b of the ion generating unit may be directed upward.

In this case, when the ion generating unit is replaced, for example, an ion generating unit replacement time button may be provided in a remote controller or the like so that when the button is pushed, the auxiliary louver 30 is operated and the upper casing 42b of the ion generating unit is directed downward.

The control section controls driving of the louver motor so that the auxiliary louver 30 is placed in a downward position with an angle with respect to the flow of the wind W to change a direction of wind in an upwardly opened position where a lower end of the wind guide panel 20 is opened forward around the upper pivot 23. Also, the control section controls driving of the louver motor so that the auxiliary louver 30 is directed in parallel with the flow of the wind W in a downwardly opened position where an upper end of the wind guide panel 20 is opened downward around the lower pivot 22.

More specifically, in the upwardly opened position of the wind guide panel 20, the control section controls the position of the auxiliary louver 30 so that the auxiliary louver 30 closes the front side of the air outlet 5, and holds air blown out forward and guides the air downward.

Figure 4 shows an example in which the auxiliary louver 30 is directed with an angle with respect to the flow of the wind in the upwardly opened position of the wind guide panel. As shown, when the auxiliary louver 30 is placed in the downward position with respect to the wind W blown out forward from the air outlet 5 through the air passage 6, the wind W directly blows against a lower surface of the auxiliary louver 30, that is, a surface to which the ion generating unit 40 is mounted. At this time, when the wind W directly blows against the ion generating element, most of the ions may be pressed against the surface of the auxiliary louver 30 and disappear.

However, an upper portion of the ion generating element 41 is covered with the upper casing 42b as shown in Figure 8. Thus, the wind W does not directly blow against the ion generating element. Meanwhile, the wind W having blown against the auxiliary louver 30 is changed in direction and introduced into the ventilation path 57, and presses out the ions generated in the ventilation path 57 toward a downstream side along the surface of the auxiliary louver 30. This allows the positive and negative ions to be introduced into the wind W and efficiently released to a distance.

Figure 3 shows an example in which the auxiliary louver 30 is directed in parallel with the flow of the wind W in the downwardly opened position of the wind guide panel. In this example, the wind guide panel 20 is pivoted downward around the lower pivot 22 to a substantially horizontal position. Specifically, in the downwardly opened position, the wind guide panel 20 is connected to the lower wall of the air outlet 5, and the wind guide panel 20 and the upper wall 5a of the air outlet 5 constitute a long nozzle. Thus, the wind guide panel 20 can guide the wind obliquely upward, and blow out the wind along the ceiling to a distance. At this time, the auxiliary louver 30 is directed in parallel with the flow of the wind W, and thus the wind W is introduced into the ventilation path 57 without changing a direction, and acts to guide the air blown out from the air outlet 5 to a distance together with ions.

In this embodiment, the protrusion on the guide mechanism is formed in the ion generating unit, and the guide section is formed in the recess, but not limited to this, the protrusion may be formed in the recess, and the guide section may be formed in the ion generating unit.

In this embodiment, the separate air conditioner is used as an air conditioner for description, but the present invention may be applied to a wind direction changing plate of an air cleaner, or a wind direction changing plate of a cool air fan that blows out cool air using vaporization heat.

### Industrial Applicability

The present invention can be conveniently used as an air-conditioning device (air blower, air conditioner, air cleaner, humidifier, dehumidifier, cooler, heater, or the like) that can blow out ions together with air from an air outlet.

## Claims

1. An air-conditioning device comprising:
a wind direction changing plate provided in an air outlet that blows out air; and
an ion generating unit detachably mounted to the wind direction changing plate,
wherein a recess is formed in the wind direction changing plate, and the ion generating unit is mounted so as to be fitted in the recess.

2. The air-conditioning device according to claim 1, wherein the recess is formed to be larger than the ion generating unit in one direction so that the ion generating unit can be fitted in the recess only in a predetermined position away from one of opposite ends of the recess in one direction, a guide mechanism is provided that slidably holds the ion generating unit fitted in the recess, and the guide mechanism slides the ion generating unit fitted in the recess toward one end in one direction to secure the ion generating unit to the wind direction changing plate.

3. The air-conditioning device according to claim 2, wherein the ion generating unit fitted in the recess is slid toward one end in one direction, and a movement regulating member is inserted in a space created between an inner surface on the other end of the recess in one direction and the ion generating unit to regulate sliding of the ion generating unit.

4. The air-conditioning device according to claim 3, wherein the movement regulating member is provided so as to be pivotably switched between an insertion position for regulating movement of the ion generating unit and a release position for releasing regulation of the movement of the ion generating unit.

5. The air-conditioning device according to claim 2, wherein the guide mechanism includes a protrusion formed on either opposite side surfaces of the ion generating unit in one direction or opposite inner surfaces of the recess in one direction, and a guide section that is formed on the other and slidably engages the protrusion, the guide section includes a lateral guiding path provided in the one direction, and a vertical guiding path having one end connected to a midpoint of the lateral guiding path perpendicularly to the lateral guiding path, and the other end opening in a peripheral edge of the recess, the protrusion can be introduced through the vertical guiding path into the lateral guiding path, and when the ion generating unit is moved from one end toward the other end of the recess in one direction to abut against the other end with the protrusion being introduced in the lateral guiding path, the protrusion passes over the vertical guiding path, and stops in the lateral guiding path on an opposite side.

6. The air-conditioning device according to claim 2, wherein a pair of connectors that electrically connect the wind direction changing plate and the ion generating unit are formed on one end surface of the ion generating unit in one direction and an inner surface on one end of the recess in one direction, the connector includes a male connector and a female connector, and when the ion generating unit is slid toward one end of the recess in one direction, the male connector engages the female connector.

7. The air-conditioning device according to claim 6, wherein the male connector is formed on one end surface of the ion generating unit in one direction, and an electrode terminal is formed on a bottom surface of the male connector.

8. The air-conditioning device according to claim 7, wherein a protective cover that prevents a finger from entering the male connector is formed on a top surface of the male connector.

9. The air-conditioning device according to claim 2, wherein positioning indications are provided on both the ion generating unit and the recess so as to face each other when the ion generating unit is placed in a position to be fitted in the recess.

10. The air-conditioning device according to claim 2, wherein the ion generating unit includes a casing, and an ion generating element housed in the casing, the casing includes a lower casing housed in the recess with the ion generating unit being mounted to the wind direction changing plate, and an upper casing protruding from a surface of the wind direction changing plate and having a space therein, the upper casing has openings on upstream and downstream sides in accordance with a direction of wind flowing along the surface of the wind direction changing plate, and the ion generating element generates ions in the space in the upper casing.
